# EUROPEAN PATENT APPLICATION

(11) **EP 3 995 132 A1**
(43) Date of publication of application: **11.05.2022**
(21) Application number: 21196268.3
(22) Date of filing: 13.09.2021
(51) Int. Cl.: A61K 9/08, A61K 31/573, A61K 31/18, A61K 31/167, A61K 47/02

(54) **DENTAL LOCAL ANESTHETIC SOLUTION**

(30) Priority: 06.11.2020 JP 2020185536
(71) Applicant: Showakai Medical Co., Saga shi, Saga 840-0201 (JP)
(72) Inventor: Hattori, Koji, Saga, 840-0201 (JP)
(74) Representative: dompatent von Kreisler Selting Werner - Partnerschaft von Patent- und Rechtsanwälten mbB

(57) **Abstract**

The main subject of the present invention is to provide a novel dental local anesthetic solution which, when administered to an affected area in the oral cavity, can reduce unidentified complaints of a patient, especially discomfort around the oral cavity, that can be evoked after treatment.
The present invention can include, for example, a dental local anesthetic injection solution, consisting of a local anesthetic; a steroid drug; an additive; water; and optionally, a vasoconstrictor, wherein the dental local anesthetic injection solution is administered near an affected area in the oral cavity prior to dental treatment.

According to the present invention, it is possible to reduce discomfort around the oral cavity after dental treatment in patients who have undergone local anesthesia for the treatment.

## Description

### TECHNICAL FIELD

The present invention pertains to the technical field of pharmaceutical preparations. The present invention relates to a dental local anesthetic solution containing a steroid drug in a local anesthetic.

### BACKGROUND OF THE INVENTION

Local anesthetics are usually injected into the vicinity of an affected area in the oral cavity, such as when shaving teeth or performing surgery on teeth. Moreover, local anesthetics are sometimes injected into an area where nerves exist to anesthetize the tip of the area where nerves are located. These methods of anesthesia are referred to as infiltration anesthesia and conduction anesthesia, respectively. In any case, patients often feel discomfort around the oral cavity after treatment.

Many dental local anesthetics contain, in addition to anesthetics such as lidocaine, vasoconstrictors in order to prevent anesthetics from being transferred to peripheral blood vessels and eliminated, and to prolong the action time (for example, see Non-Patent Document 1). Such vasoconstrictors can include, for example, epinephrine (adrenaline). In addition, many dental local anesthetics contain preservatives such as parabens and additives such as pH adjusters.

Dental local anesthetics used in infiltration anesthesia are, in Japan, usually provided in a cartridge type containing 1 mL or 1.8 mL of drug solution. This cartridge is loaded into a dedicated syringe and, for example, an injection needle is attached to the syringe and used. Local anesthesia is performed manually or electrically.

Steroids are adrenocortical hormones that have effects such as suppressing inflammation, immunity, and allergy, and are widely used in various medical treatments. In addition to oral medicines such as tablets and external preparations such as ointments, steroids have also been provided as injection solutions (for example, Non-Patent Document 2).

### PRIOR ART DOCUMENTS

### NON-PATENT DOCUMENT

Non-Patent Document 1: Pharmaceuticals and Medical Devices Agency, "Dental Xylocaine Cartridge," revised in January 2017 (13th edition), [on line], [Search on October 9, 2020], Internet (https://www.info.pmda.go.jp/go/pack/2710806U1021_3_07/)
Non-Patent Document 2: Pharmaceuticals and Medical Devices Agency, "Decadron Injection 1.65 mg," revised in July 2020 (2nd edition), [on line], [Search on October 9, 2020], Internet (https://www.info.pmda.go.jp/go/pack/2454405H1024_3_04/)

### SUMMARY OF THE INVENTION

### PROBLEM TO BE SOLVED BY THE INVENTION

The present invention is primarily directed to provide a novel dental local anesthetic solution which, when administered to the vicinity of an affected area in the oral cavity, can reduce unidentified complaints of patients (adults, children) that may be caused after treatment, particularly discomfort around the oral cavity.

### MEANS FOR SOLVING THE PROBLEMS

As a result of intensive studies, the present inventor has found that the above problem can be solved luckily by adding a steroid drug in a commercially available local anesthetic, and the present invention was completed.

The present invention can include, for example, the following embodiments.
[1] A dental local anesthetic solution, comprising an effective amount of a local anesthetic and an effective amount of a steroid drug as active ingredients in an aqueous solution.
[2] The dental local anesthetic solution according to [1] above, which is prepared by removing a part of the solution of a dental local anesthetic preparation containing a local anesthetic as an active ingredient, and adding a steroid injection solution containing a steroid drug as an active ingredient into the dental local anesthetic preparation solution remaining after the removal.
[3] The dental local anesthetic solution according to [1] or [2] above, which is contained in a cartridge, contained in a cartridge which is loaded into a syringe, or contained in a cartridge which is loaded into a syringe to which an injection needle is attached.
[4] The dental local anesthetic solution according to any one of [1] to [3] above, further comprising a vasoconstrictor.
[5] The dental local anesthetic solution according to any one of [1] to [4] above, wherein the local anesthetic is lidocaine or a pharmaceutically acceptable salt thereof, and wherein the steroid drug is dexamethasone, an ester thereof, or a pharmaceutically acceptable salt thereof.
[6] A dental local anesthetic solution containing a local anesthetic as an active ingredient for use in combination with a steroid drug.
[7] A process for producing a dental local anesthetic solution, comprising a step of removing a part of the solution of a dental local anesthetic preparation containing a local anesthetic as an active ingredient, and a step of adding a steroid injection solution containing a steroid drug as an active ingredient into the dental local anesthetic preparation solution remaining after the removal.

### EFFECT OF THE INVENTION

According to the present invention, it is possible to reduce discomfort around the oral cavity after dental treatment in patients who have undergone local anesthesia for the treatment. Such discomfort can include, for example, numbness of lips, stiffness, tingling, cold, and itching (particularly in children). Reducing such discomfort can also reduce the lip-biting or cheek-scratching behavior seen particularly after treatment of children.

Moreover, anaphylaxis and nerve irreversibility may be prevented as well as the above-mentioned discomfort around the oral cavity.

### BRIEF DESCRIPTION OF THE DRAWINGS

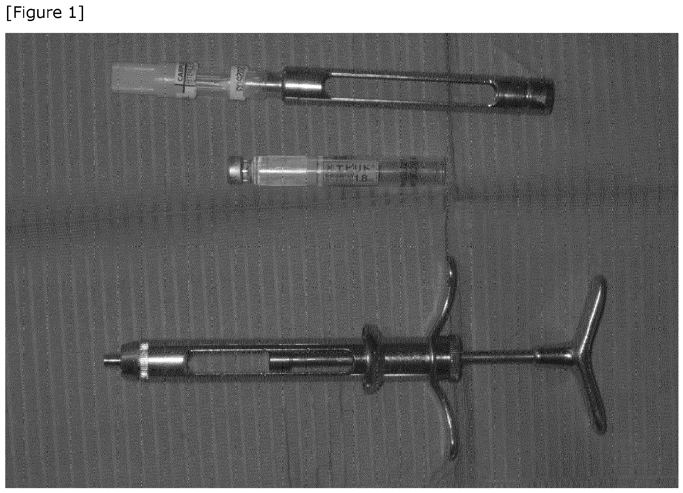

Figure 1 is a photograph representing an example of a cartridge type of a dental local anesthetic (center) and an anesthetic instrument for use in using the dental local anesthetic.

### EMBODIMENT FOR CARRYING OUT THE PRESENT INVENTION

### 1 Local anesthetic solution according to the present invention

The local anesthetic solution according to the present invention (hereinafter, referred to as "the local anesthetic solution of the present invention") is a local anesthetic injection solution for administration to the vicinity of an affected area in the oral cavity prior to dental treatment, comprising an effective amount of a local anesthetic and an effective amount of a steroid drug as active ingredients in an aqueous solution. The local anesthetic solution of the present invention may be a local anesthetic solution, which is prepared by removing a part of the solution of a dental local anesthetic preparation containing a local anesthetic as an active ingredient, and adding a steroid injection solution containing a steroid drug as an active ingredient into the dental local anesthetic preparation solution remaining after the removal. Here, the amount of the steroid injection solution to be added may be the same amount as or different from the amount of the local anesthetic solution to be removed.

Moreover, the local anesthetic solution of the present invention may be a dental local anesthetic solution containing a local anesthetic as an active ingredient for use in combination with a steroid drug. In the case of the local anesthetic solution of the present invention, the dental local anesthetic solution may be present independently of the steroid drug, and the steroid drug may not necessarily be included.

### 1.1 Local anesthetics

The local anesthetic solution of the present invention contains a local anesthetic as an active ingredient. The local anesthetic is not particularly limited so long as it is what is called a sodium channel blocker which can be used in dental treatment and is soluble in water, and which may either be a short-acting type or a long-acting type. Specifically, it can include the ester type such as procaine and tetracaine, and the amide type such as lidocaine, mepivacaine, bupivacaine, levobupivacaine, ropivacaine, and propitocaine. Among them, amide type local anesthetics are preferable, and especially lidocaine, mepivacaine, and propitocaine are preferable. These are usually provided in the form of a pharmaceutically acceptable salt in order of increasing solubility in water. The pharmaceutically acceptable salt can include, for example, an inorganic acid salt such as hydrochloride, sulfate and phosphate; and an organic acid salt such as oxalate, citrate, lactate, succinate and malate. When the local anesthetic is a salt, lidocaine hydrochloride and mepivacaine hydrochloride are preferred. A local anesthetic preparation containing lidocaine hydrochloride is sold under the trade name of, for example, Xylocaine (registered trademark) Cartridge for Dental Use, ORA (registered trademark) Inj. Dental Cartridge, Epilido (registered trademark) Cartridge. A local anesthetic preparation containing mepivacaine hydrochloride is sold under the trade name of, for example, Scandonest (registered trademark) cartridge. A local anesthetic preparation containing propitocaine hydrochloride salt is sold under the trade name, for example, Citanest-Octapressin (registered trademark) Cartridge for Dental Use. The volume of these commercial products is 1 mL or 1.8 mL. Either of them can be used in the local anesthetic solution of the present invention.

The content of the local anesthetic in the local anesthetic solution of the present invention varies depending on the kind of the local anesthetic, other components, and the like, and is suitably within the range of 12 to 40 mg/mL, preferably within the range of 14 to 35 mg/mL, and more preferably within the range of 16 to 30 mg/mL. In case that the local anesthetic is lidocaine, it is suitable within the range of 12 to 25 mg/mL, preferably within the range of 14 to 22 mg/mL, and more preferably within the range of 16 to 20 mg/mL. The content smaller than 12 mg/mL or larger than 40 mg/mL is not excluded, but the former may require a large amount of administration to exert an anesthetic effect, and the latter may cause insufficient dissolution depending on the kind of the local anesthetic.

### 1.2 Steroid drugs

The local anesthetic solution of the present invention contains a steroid drug as an active ingredient. The steroid drug is not particularly limited so long as it is an adrenocortical hormone which can be used in dental treatment and is soluble in water. Moreover, it may be a short-acting type (half-life: 8 to 12 hours), a medium-acting type (half-life: 12 to 36 hours), or a long-acting type (half-life: 36 to 54 hours). Specifically, the active substance of the steroid drug can include, for example, hydrocortisone (cortisol), cortisone, prednisolone, methylprednisolone, triamcinolone, dexamethasone, betamethasone, and their esters. The ester can include, for example, phosphate ester, succinate ester, acetate ester, palmitate ester. Moreover, they are usually provided in the form of a pharmaceutically acceptable salt in order of increasing solubility in water. The pharmaceutically acceptable salt can include, for example, an alkali metal salt such as sodium salt and potassium salt; and an alkaline earth metal salt such as magnesium salt and calcium salt. Among them, dexamethasone, an ester thereof or a salt thereof is preferred, and sodium dexamethasone phosphate ester is more preferred.

The content of the steroid drug in the local anesthetic solution of the present invention varies depending on the kind of the steroid drug, the kind of the local anesthetic, other components, and the like, and is suitably within the range of 1.2 to 16 mg/mL, preferably within the range of 1.4 to 14 mg/mL, and more preferably within the range of 1.6 to 12 mg/mL. In case that the steroid drug is dexamethasone, it is suitable within the range of 0.05 to 1 mg/mL, preferably within the range of 0.1 to 0.7 mg/mL, and more preferably within the range of 0.3 to 0.6 mg/mL. If the content is smaller than 0.05 mg/mL, the effect of the present invention may not be sufficiently obtained.

Basically, the content may be small.

### 1.3 Vasoconstrictors

The local anesthetic solution of the present invention may include a vasoconstrictor as an active ingredient. The vasoconstrictor is usually added to facilitate local stagnation of the local anesthetic and prolong its duration of action. Specific examples of the vasoconstrictor can include epinephrine (adrenaline), a pharmaceutically acceptable salt thereof (e.g., hydrogen tartrate), and felypressin.

The content of the vasoconstrictor in the local anesthetic solution of the present invention varies depending on the kind of the local anesthetic, other components, and the like, and is suitably within the range of 0.005 to 0.1 mg/mL, preferably within the range of 0.008 to 0.05 mg/mL, and more preferably within the range of 0.01 to 0.02 mg/mL. In case that the vasoconstrictor is epinephrine, the same content as described above can be applicable.

### 1.4 Other components

The local anesthetic solution of the present invention may contain an appropriate amount of an additive such as a pharmaceutically acceptable preservative, a pharmaceutically acceptable pH adjuster, a pharmaceutically acceptable isotonizing agent, a pharmaceutically acceptable dissolution aid, and the like, if necessary.

Examples of the preservative can include sodium bisulfite, sodium pyrosulfite, methyl paraoxybenzoate, ethyl paraoxybenzoate, and propyl parahydroxybenzoate.

Examples of the pH adjuster can include lactic acid, carbon dioxide, succinic acid, gluconic acid, citric acid, trisodium citrate, phosphoric acid, potassium carbonate, and sodium bicarbonate.

Examples of the isotonizing agent can include sodium chloride, glycerin, and glucose.

Examples of the dissolution aid can include propylene glycol and polyethylene glycol.

The local anesthetic solution of the present invention is preferably contained in a suitable cartridge for dental local anesthesia, for example, as shown in the center of Figure 1. The cartridge may also be loaded into a dedicated syringe, to which an injection needle or the like may further be attached. A kit comprising a cartridge in which the local anesthetic solution of the present invention is contained, a syringe, and an injection needle or the like can also be included in the present invention.

### 2 Processes for producing and using the local anesthetic solution of the present invention

The local anesthetic solution of the present invention can be produced, for example, as follows. The local anesthetic solution of the present invention can be produced by removing a part of the solution of a dental local anesthetic preparation containing a local anesthetic as an active ingredient, and adding a steroid injection solution containing a steroid drug as an active ingredient into the local anesthetic preparation solution remaining after the removal. It can also be produced by simply adding a steroid injection solution into a local anesthetic preparation solution without removing a part of the solution of the local anesthetic preparation. Alternatively, the local anesthetic solution of the present invention can be produced in a process comprising a step of removing a part of the solution of a dental local anesthetic preparation containing a local anesthetic as an active ingredient, and a step of adding a steroid injection solution containing a steroid drug as an active ingredient into the local anesthetic preparation solution remaining after the removal. Moreover, for example, the local anesthetic solution of the present invention can be produced by dissolving a local anesthetic and a steroid drug mentioned above, and, if necessary, a vasoconstrictor mentioned above and a desired additive in water such as water for injection, purified water, or distilled water so that the above-mentioned contents are attained by a conventional method.

The local anesthetics, steroid drugs, vasoconstrictors, additives, and the like are the same meaning as mentioned above.

The above-mentioned dental local anesthetic preparation containing a local anesthetic can include, for example, a commercial cartridge-type dental local anesthetic (1 mL or 1.8 mL). A commercially available cartridge-type dental local anesthetic can be used as it is. In this case, the cartridge used in the commercial product can be applied as it is. The dental local anesthetic preparation can include, for example, the above-mentioned Xylocaine (registered trademark) Cartridge for Dental Use, Ora (registered trademark) Inj. Dental Cartridge, Epilido (registered trademark) Cartridge, Scandonest (registered trademark) cartridge, and Citanest-Octapressin (registered trademark) Cartridge for Dental Use.

The above-mentioned steroid injection containing a steroid drug can include, for example, a commercially available steroid injection which can be used for dental treatment. A commercial steroid injection can be used as it is. The steroid injection can include, for example, Water-soluble Hydrocortone (registered trademark) injection (containing sodium hydrocortisone phosphate), Solu-Cortef (registered trademark) injection (containing sodium hydrocortisone succinate), Saxizon (registered trademark) for injection (containing sodium hydrocortisone succinate), Water-soluble Predonine (registered trademark) for injection (containing sodium prednisolone succinate), Solu-Medrol (registered trademark) for injection (containing sodium methylprednisolone succinate), Depo-Medrol (registered trademark) for injection (containing methylprednisolone acetate), Decadron (registered trademark) for injection (containing dexamethasone sodium phosphate), Orgadrone (registered trademark) injection (containing dexamethasone sodium phosphate), Limethasone (registered trademark) for injection (containing dexamethasone palmitate), and Rinderon (registered trademark) for injections (containing sodium betamethasone phosphate) .

As the amount of liquid to be removed when removing a part of the solution of a dental local anesthetic preparation, for example, in case that it is removed from a commercially available cartridge-type dental local anesthetic preparation, it is suitably within the range of 1 to 60% of the total volume, preferably within the range of 5 to 50%, and more preferably within the range of 10 to 45%.

The amount of a steroid injection solution to be added varies depending on the kinds of the local anesthetic preparation and the steroid injection to be used, and the like, and the amount is, for example, suitably within the range of 10 to 150% of the amount of the removed local anesthetic preparation solution, preferably within the range of 20 to 100%, and more preferably within the range of 30 to 60%. If necessary, it may be less than 10%, or alternatively more than 150%.

The local anesthetic solution of the present invention can be, for example, contained in a suitable cartridge for dental local anesthesia by a conventional method. Moreover, a cartridge in which the local anesthetic solution of the present invention is contained can be loaded into a dedicated syringe. Further, an injection needle or the like can be attached to the syringe.

The local anesthetic solution of the present invention can be used in the same manner as, for example, a commercially available cartridge-type dental local anesthetic preparation, using a commercially available manual or electrically-powered anesthetic instrument. Specifically, it can be used by injecting manually or electrically a suitable amount within the range of 0.8 mL to 2 mL (preferably 1 mL to 1.8 mL) into the vicinity of an affected area in the oral cavity, in the form of being contained in a suitable cartridge which is loaded into a dedicated syringe to which an injection needle is attached. Note that the local anesthetic solution of the present invention can be used for anyone from adults to children, and regardless of sex or age (young or elderly).

The needle is not particularly limited so long as it is used for dental treatment, and the thickness of the needle may be, for example, around 30 gauge (G), specifically, within the range of 26 to 32 G, and is preferably within the range of 28 to 31 G.

In case that the local anesthetic solution of the present invention is a dental local anesthetic solution containing a local anesthetic as an active ingredient for use in a combination with a steroid drug, for example, the local anesthetic solution of the present invention, which does not contain a steroid drug, may be injected in an appropriate amount into the vicinity of an affected area to be treated in the oral cavity, and then a steroid injection solution containing a steroid drug as an active ingredient may be injected into the vicinity of the affected area. Moreover, the local anesthetic solution of the present invention, which does not contain a steroid drug, may be injected in an appropriate amount into an affected area to be treated in the oral cavity, and then a steroid injection solution containing a steroid drug as an active ingredient may be injected into the vicinity of the affected area, followed by injecting again the local anesthetic solution of the present invention, which does not contain a steroid drug, in an appropriate amount into the vicinity of the affected area.

For the above use, the local anesthetic solution of the present invention, which does not contain a steroid drug, and a steroid injection solution can also be provided as a kit or the like, and such a kit or the like is also included in the present invention.

### EXAMPLE

Hereinafter, the present invention will be illustrated with reference to Test Examples, Examples, and the like, but the present invention is not limited to these examples and the like.

### [Example 1] Preparation of the local anesthetic solution of the present invention (1)

A solution of about 0.8 mL of the commercially available Epilido (registered trademark) Cartrige 1.8 mL was removed by a disposable syringe, and a solution of about 0.3 mL of Decadron (registered trademark) Injection 3.3 mg was added to the local anesthetic solution of about 1 mL remaining in the cartridge to prepare the local anesthetic solution of the present invention. The local anesthetic solution of the present invention is contained in the original cartridge of the Epilido (registered trademark) Injection.

### [Example 2] Preparation of the local anesthetic solution of the present invention (2)

The local anesthetic solution of the present invention was prepared in the same manner as in Example 1, using Scandonest (registered trademark) cartridge 3%, which is a dental local anesthetic preparation, instead of Epilido (registered trademark) Cartrige 1.8 mL.

### [Example 3] Preparation of the local anesthetic solution of the present invention (3)

The local anesthetic solution of the present invention was prepared in the same manner as in Example 1, using Citanest-Octapressin (registered trademark) Cartridge instead of Epilido (registered trademark) Cartrige 1.8 mL.

### [Test Example 1] Verification of the effect of reducing discomfort in patients after treatment (1)

The local anesthetic solution of the present invention of Example 1, contained in a cartridge, was loaded into a dedicated anesthetic syringe attached with a 1 G, 33 G or 35 G needle and injected into a patient's left gingiva. On the other hand, a control local anesthetic solution prepared by simply removing the same amount as above from Epilido (registered trademark) injection solution, which had not been supplemented with Decadron (registered trademark) injection, was similarly injected into the patient's right gingiva almost at the same time.

As a result, the effects of anesthesia near the injection sites of left and right gingiva were similar, but in the vicinity of the left gingiva to which the local anesthetic solution of the present invention containing Decadrone (steroid drug) was injected, the anesthesia disappeared sometime unconsciously, and in the meanwhile no patient complained of discomfort such as numbness of the lip. On the other hand, in the vicinity of the right gingiva to which the conventional local anesthetic solution, that is the above control local anesthetic solution, without Decadron (steroid drug) was injected, most of patients complained that discomfort such as numbness of the lip did not disappear even after 3 to 4 hours.

Moreover, when the local anesthetic solution of the present invention containing Decadrone (steroid drug) was separately injected into the vicinity of the affected area (about 300 persons), the anesthesia disappeared sometime unconsciously, and in the meanwhile no patient complained of discomfort such as numbness of the lip. Similar results were obtained for the local anesthetic solution of the present invention according to Examples 2 and 3.

From the above, it is apparent that the local anesthetic solution of the present invention alleviates discomfort around the oral cavity after dental treatment of a patient.

### [Test Example 2] Verification of the effect of reducing discomfort in patients after treatment (2)

In ten patients receiving a dental treatment, only 0.8 mL of Citanest-Octapressin (registered trademark, propitokine injection) or Scandonest (registered trademark, mepivacaine injection) was administered to the left buccal region (in the left gingiva) and, to the right buccal region (in the right gingiva), the local anesthetic solution of the present invention containing the same local anesthetic preparation in combination with 0.4 mL (100 mg/2 mL) of Solu-Cortef (registered trademark, hydrocortisone) was administered.

As a result, in all the patients, the left buccal region to which only the local anesthetic was administered remained uncomfortable (discomfort, or being about to bite the lips, etc.) for a long time even after the anesthesia worn off. Whereas, the right buccal region to which the local anesthetic and hydrocortisone (steroid drug) were administered in combination did not remained uncomfortable like the left buccal region. All the patients stated their impression that the local anesthetic solution of the present invention containing hydrocortisone (steroid drug) was superior.

### [Industrial Applicability]

The local anesthetic solution of the present invention is useful in the treatment of a dental region or the like, because it can reduce unidentified complaints of a patient after dental treatment, in particular, discomfort around the oral cavity.

## Claims

1. A dental local anesthetic injection solution for administering near an affected area in the oral cavity prior to dental treatment, consisting of
(1) one or more selected from the group consisting of lidocaine, mepivacaine, propitocaine, and their pharmaceutically acceptable salts;
(2) one or more selected from the group consisting of hydrocortisone, prednisolone, methylprednisolone, triamcinolone, dexamethasone, betamethasone, their esters, and their pharmaceutically acceptable salts;
(3) an additive;
(4) water; and
(5) optionally, a vasoconstrictor.

2. The dental local anesthetic injection solution according to Claim 1, which is contained in a cartridge, contained in a cartridge which is loaded into a syringe, or contained in a cartridge which is loaded into a syringe to which an injection needle is attached.

3. The dental local anesthetic injection solution according to Claim 1 or 2, wherein the additive is one or more selected from the group consisting of a preservative, a pH adjuster, an isotonizing agent, and a dissolution aid.

4. The dental local anesthetic injection solution according to any one of Claims 1 to 3, wherein the local anesthetic is lidocaine or a pharmaceutically acceptable salt thereof, and the steroid drug is dexamethasone, an ester thereof or a pharmaceutically acceptable salt thereof.

5. The dental local anesthetic injection solution according to any one of Claims 1 to 4, wherein the vasoconstrictor is epinephrine or a pharmaceutically acceptable salt thereof, or felypressin.

6. A process for producing a dental local anesthetic injection solution, comprising a step of removing a part of the solution of a dental local anesthetic preparation containing as an active ingredient(s) one or more selected from the group consisting of lidocaine, mepivacaine, propitocaine, and their pharmaceutically acceptable salts; and a step of adding a steroid injection solution containing as an active ingredient(s) one or more selected from the group consisting of hydrocortisone, prednisolone, methylprednisolone, triamcinolone, dexamethasone, betamethasone, their esters, and their pharmaceutically acceptable salts into the dental local anesthetic preparation solution remaining after the removal.
